# EUROPEAN PATENT APPLICATION

(11) **EP 3 290 438 A1**
(43) Date of publication of application: **07.03.2018**
(21) Application number: 16382413.9
(22) Date of filing: 06.09.2016
(51) Int. Cl.: C07K 14/46, C07K 14/76

(54) **FOOD ALLERGEN EXTRACTS AND METHODS OF PRODUCING AND USING THE SAME**

(71) Applicant: CONSEJO SUPERIOR DE INVESTIGACIONES CIENTIFICAS, 28006 Madrid (ES); Fundación para la Investigación Biomédica del Hospital Universitario de la Paz, 28046 Madrid (ES)
(72) Inventor: Sánchez Herreros, Rosa, 28006 Madrid (ES); Martínez Fernández, Javier, 28006 Madrid (ES); Castro Morera, Ana, 28006 Madrid (ES); Gasset Vega, María, 28006 Madrid (ES); Rodríguez Pérez, Rosa M, 28046 Madrid (ES); Pedrosa Delgado, María, 28046 Madrid (ES); Quirce Gancedo, Santiago, 28046 Madrid (ES)
(74) Representative: Pons

(57) **Abstract**

The present invention refers to a method for producing improved food allergen extracts, the food allergen extracts produced and their use for the diagnosis of type I food allergy. Particularly, the food allergen extracts of the invention comprise a type I food allergen in an amyloid and insoluble state which strengths its interaction with the IgE present in the patient serum, allowing thus to diagnose the food allergy in a more reliable manner, reducing the number of false negatives obtained by other diagnosis methods that use monomeric food allergens.

## Description

This invention belongs to the field of immunology and food allergy diagnosis methods. Specifically, it refers to a method for producing improved food allergen extracts, the food allergen extracts produced and their use for the diagnosis of type I food allergy.

### BACKGROUND ART

Allergy is an acquired hypersensitivity disorder of the immune system and is triggered by exposure to harmless environmental substances known as allergens. A type I hypersensitivity reaction is characteristic of allergic reactions and results in the production of excessive amounts of IgE antibodies which in turn activate basophils and mast cells causing an inflammatory reaction. The effects may be systemic such as vasodilation, mucus secretion, nerve stimulation and smooth muscle contraction causing an anaphylaxis reaction, or confined to a particular area of the body, for example the respiratory system.

Food allergy is an emerging major public health problem that affects around 6% of school children and approximately 4% of adults and can have severe consequences, including fatal anaphylactic reactions. Allergy can therefore have a significant impact on psychosocial aspects of quality of life extending beyond the immediate clinical effects of the patient's allergic condition and the daily activities of families. At present the standard of care for this type of allergy includes strict avoidance of the offending allergens and treatment with epinephrine.

Type I food allergy, such as fish allergy, is a hypersensitivity reaction in which IgE is involved. The intake of the allergen in hypersensitive patients leads to a symptomatology that may be lethal. Tests in the skin of the patient, together with the medical record, are the main diagnosis tools. These tests, named Skin Prick Tests (SPT), asses the IgE reactivity linked to the patient mast cells. The skin test consists of eliciting a reaction in the patient skin by adding a small allergen amount. Thus, a small allergen amount is deposited in the arm of the patient, afterwards an epidermis puncture is made so that the allergen is allowed to infiltrate in deeper layers of the skin. If the patient is allergic, a histamine release from the skin mast cells will be observed as a consequence of the allergic response, and inflammation and redness will be detected. Reactives used in these SPT diagnosis methods are extracts containing standardized and diluted allergens Besides the contact with the allergen, positive (an histamine solution) and negative (a saline solution) controls are usually performed.

Additionally, a definitive test for the diagnosis of food allergies can be also performed, which consists of the oral exposure to the causal food (Oral Food Challenge). However, this test is highly risky.

There is, therefore, a need to further improve the efficacy of allergy diagnosis methods to ensure that allergic patients are properly identified, eliminating or lowering the number of false negatives detected with current diagnosis methods. In this sense, molecular basis of the interactions between the allergen and IgE should be studied further in order to design improved allergen extracts capable of increasing their interaction with the IgE. These improved allergen extracts would allow a more reliable diagnosis when used in SPT solutions as a reactive.

Amyloids are polymeric structural protein states formed from locally or totally unfolded protein chains that permit surface reorganizations, stability enhancements and interaction properties that are absent in the precursor monomers.

Amyloids are defined by a cross-β sheet backbone, in which β-sheets that are mostly formed from β-strands of different molecules pack through their side chains (Goldschmidt, L., et al. Proceedings of the National Academy of Sciences of the United States of America 107, 3487-3492, (2010)). Despite their initial association with proteins involved in neurodegenerative disorders, an increasing number of proteins that form functional and pathogenic amyloids have been reported. Indeed, protein chains containing sequences with cross-β sheet folding traits under conditions that cause their solvent exposure enter this state when they are present at sufficient concentrations to overcome the entropy that opposes fibril ordering. Amyloids exhibit a dual (soluble/insoluble) nature and a characteristic structural repetitiveness, allowing phase separations and interactions with enhanced affinity and specificity compared to the precursor monomer (Greenwald, J. & Riek, R. Structure 18, 1244-1260, (2010)).

Fish β-parvalbumins represent the major allergens of IgE-mediated fish hypersensitive patients (Sharp, M. F. & Lopata, A. L. Clinical reviews in allergy & immunology 46, 258-271, (2014)). β-Parvalbumins are 12 kDa calcium-binding proteins with three EF hand motifs; a nonfunctional AB motif, followed by the CD and EF Ca²⁺-binding motifs (Berchtold, M. W., Brinkmeier, H. & Muntener, M. Physiological reviews 80, 1215-1265 (2000)). Despite the variations in sequence identity between proteins from the same and different fish species, β-parvalbumins display a high IgE cross-reactivity, supporting a pattern of recognition other than the sequence identity (Van Do, T., et al. J Allergy Clin Immun 116, 1314-1320, (2005)). The use of a variety of approaches, including protease digests, phage display libraries, site-directed mutagenesis and arrays of overlapping peptides with β-parvalbumins from *Baltic cod* (Gad c 1) (Lapteva, Y. S., et al. Bba-Proteins Proteom 1834, 1607-1614, (2013)), *Atlantic cod* (Gad m 1) (Perez-Gordo, M. et al. Mol Nutr Food Res 57, 1283-1290, (2013)), *Common carp* (Cyp c 1) (Swoboda, I. et al. J Immunol 178, 6290-6296 (2007)), *Chub mackerel* (Sco j 1) (Yoshida, S., Ichimura, A. & Shiomi, K. Food Chem 111, 857-861, (2008)) and Atlantic salmon (Sal s 1) (Perez-Gordo, M. et al. Int Arch Allergy Imm 157, 31-40, (2012)), has shown distinct IgE epitopes of both linear and conformational types. For Gad c 1 and Sal s 1, the IgE binding sites have been located at the spacers between the AB and CD motifs (residues 28-45) and CD and EF motifs (residues 65-74), whereas for Gad m1, the dominant IgE binding region was found at the C-terminus (segment 95-109) (Perez-Gordo, M. et al. Int Arch Allergy Imm 157, 31-40, (2012)). On the other hand, disruption of the Ca²⁺ binding sites by mutagenesis in both Cyp c 1 and Sco j 1 resulted in soluble chains with reduced IgE-binding signals (Swoboda, I. et al. J Immunol 178, 6290-6296 (2007)). Conversely, stabilization of apo-Gad m 1 produces highly stable amyloids with enhanced IgE reactivity (Martinez, J. et al. Swiss Med Wkly 145, (2015)).

Despite the large number of sequences and 3D structures of allergens elucidated in the last few decades, the knowledge of how allergens recognize and cross-link cell-bound IgEs is still limited (Gieras, A. et al. The Journal of allergy and clinical immunology 119, 384-390, (2007)). This lack of knowledge is largely due to the complexity of allergen epitopes and their incomplete interpretation, based on the use of structures obtained from stable and ligand-bound states (Perez-Gordo, M. et al. Mol Nutr Food Res 57, 1283-1290, (2013)). For food allergens that undergo drastic environmental changes during gastrointestinal transit, these structural templates are merely snapshots of the protein life and other nonnative structures can be formed and change their interaction repertoire. One such structural state is the amyloid state that confers a polymeric trait required for multivalent interactions through the spine structure and growth properties. Amyloid formation is a sequence-dependent process and has been described for distinct food allergens, such as Bos d 5 (β-lactoglobulin), Bos d 10 (κ-casein), Bos d 12 (αs2-casein), Gal d 2 (ovalbumin), Gal d 4 (lysozyme) and Gad m 1 (β-parvalbumin) among others ((Martinez, J. et al. Swiss Med Wkly 145, (2015)).

In summary, improved methods for the diagnosis of type I food allergies are required, as current standardized allergen extracts used in these methods as reagents render a significant number of false negatives due to their weak interaction of the IgE present in the samples/tissues of the hypersensitive patients. Thus, improved methods for the diagnosis of type I food allergies are needed. These methods should lead to a more reliable diagnosis and should render a lower number of false negatives.

### DESCRIPTION OF THE INVENTION

The present invention provides a method for producing improved food allergen extracts, the food allergen extracts produced and their use for the diagnosis of type I food allergy.

The problem mentioned above is solved by the present invention through the use of food allergen extracts comprising a polymerized and insoluble food allergen, *i.e*. the food allergen to be used in the diagnosis method of the invention is in an amyloid state. When these food allergen extracts of the invention are used as a reactive in type I food allergy diagnosis methods the number of false negatives decreases compared to other methods in which monomeric allergens are used, as the food allergen of the invention in an amyloid state has significantly increased IgE affinity.

The amyloid fold of the food allergen extracts of the invention governs IgE binding. Food allergens, such as β-Parvalbumin which is the major allergen in fish allergy, form amyloids that are recognized by IgE in the patient sera. In the examples of the present invention, Gad m1 was used as the fish β-parvalbumin model and a combination of approaches, including peptide arrays, recombinant wt and mutant chains, biophysical characterizations, protease digestions, mass spectrometry, dot-blot and ELISA assays was performed to gain insights into the role of amyloids in the IgE interaction. It was found that Gad m1 immunoreactive regions behave as sequence-dependent conformational epitopes that provide a 1000-fold increase in affinity and the structural repetitiveness required for optimal IgE binding and cross-linking upon folding into amyloids. These findings support the amyloid state of food allergens as a key entity in type I food allergy.

Thus, in the present invention it has been addressed the role of food allergen amyloids with respect to both its IgE-reactive properties and epitope architecture. It was found that when the relevant antigenic regions of the food allergens fold into amyloids they optimize their IgE interaction, which allows for more reliable food allergy diagnosis methods.

According to the examples of the application, the allergen chains that were not in an amyloid state share a diminished IgE interaction, which leads to a less effective diagnosis method when said non-amyloid allergens are used. Both allergen monomers and amyloids bind IgE, but with significantly different affinities. The interaction of sera IgE with allergen amyloids reveals a 1000-fold enhancement of the interaction strength compared to the monomer.

From a structural point of view, the folding of the epitope into a cross-β sheet may optimize the number and nature of contacts for optimal antibody recognition. From the stoichiometry point of view, the polymer nature of amyloids permits the saturation of the two IgE binding arms, whereas a monomer does not permit saturation. The presentation of the allergen as an amyloid allows the development of a single IgE molecule and the optimization by enhancing the probability of the cross-linking step.

The phase transition accompanying amyloid formation has therefore major implications in allergy diagnosis. Clearing amyloids (either by their physical removal or by using additives that prevent their formation) from solutions used for skin prick tests may result in false negatives compared with non-risk-free oral food challenges. This strategy proposed by the present invention is of particular relevance for cases in which amyloids are preferentially formed after the partial digestion of food allergens. Therefore, in light of these results, a food allergy diagnosis should be implemented for the consideration of allergen folds displaying insolubility.

Therefore, an aspect of the present invention refers to a process for producing an allergen extract, hereinafter "the process of the invention", comprising:
a. incubating a solution comprising a type I food allergen under conditions that allow protein polymerization or protein amyloid formation, and
b. recovering the solution comprising the type I food allergen in an amyloid (or polymerized), and preferably in an insoluble, state after step (a).

An "allergen extract" may be derived from any source material comprising natural allergens known to illicit an IgE mediated immune reaction in an individual, *i.e.* the solution comprising the type I food allergen as defined in the process of the invention may be any source material comprising one or more natural type I food allergens.

A "type I food allergen" is any food protein, glycosylated or not, that produces an IgE mediated hypersensitive adverse systemic reaction via the gastrointestinal system and generally exhibits heat, low pH and protease resistance. Type I food allergen may be the complete protein sequence or a relevant fragment thereof, both isolated from natural sources or produced either recombinantly or synthetically. The "relevant fragment thereof" is preferably any immunoreactive protein fragment or any other fragment capable of folding into an amyloid state. Examples of type I food allergens are, without limitation, milk allergens such as β-lactoglobulin (Bos d 5) or caseins (Bos d 12, etc), peanut allergens (Ara h 1, Ara h 2, etc.), egg allergens such as lysozyme (Gal 4 d), ovalbumin (Gal d 2), and ovotransferrin (Gal d 3), shellfish allergens, fish allergens such β-parvalbumins (Gad m 1, Gad c 1, The c 1, Sal s 1, Cyp c 1, Car ca 1, and others in the allergome database), and nut, soy, wheat or rice allergens.

In a preferred embodiment of the process of the invention, the type I food allergen is a fish β-Parvalbumin protein, preferably the Gad m1 protein which is, for example, any sequence selected from the list consisting of: Q90YK9, Q90YL0 (UniProtKB/Swiss-Prot), A5I873, A5I874 (GeneBank) and their variants or relevant fragments.

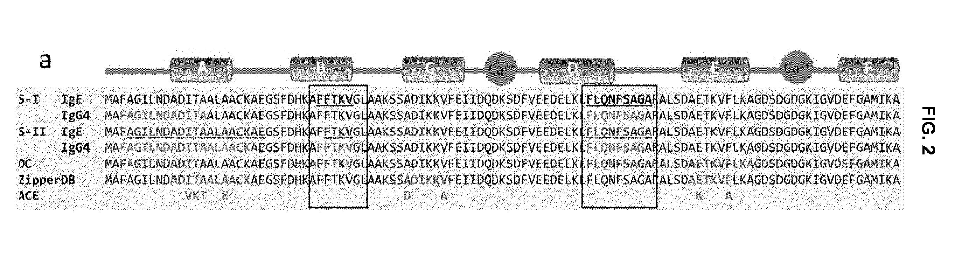

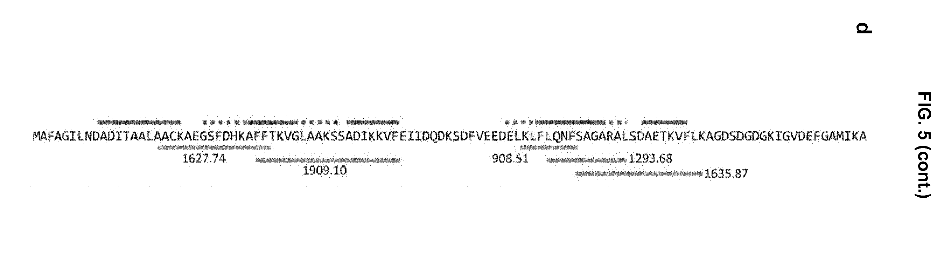

Examples of immunoreactive and amyloid forming regions in the Gad m 1 chain that can be used, alone or in combination, in the process of the invention as relevant fragments of the type I food allergen Gad m 1 are any of those identified in the Figures of the present application, specifically in Figs. 2a and 5d.

In a more preferred embodiment of the process of the invention, the conditions that allow protein polymerization or protein amyloid formation are selected from the list consisting of: the presence of EDTA, the presence of an acid pH (preferably pH<1.9), the presence of proteases (such as pepsin, chymotrypsin, trypsin or any combination thereof), temperature changes (including heating at different temperatures, changes in the heating time and the use or not of agitation processes), lipid content variations or their combinations. These conditions will depend on the type I food allergen present in the solution. When the type I food allergen is a β-Parvalbumin protein, preferably the Gad m1 protein, the conditions are the presence of EDTA and preferably an incubation time of at least 70h at 37 °C.

The "amyloid or polymerized state" is that state in which the monomeric type I food allergen molecules are grouped together so that they produce a molecule with a higher molecular weight, which includes aggregation states.

Another aspect of the invention refers to an allergen extract that comprises a type I food allergen in an amyloid (or polymerized), and preferably in an insoluble, state. Hereinafter, this allergen extract will be referred to as "allergen extract of the invention".

In a preferred embodiment, the allergen extract of the invention is that obtainable by the process of the invention.

In a more preferred embodiment, the type I food allergen comprised in the allergen extract of the invention is a β-Parvalbumin protein, preferably the Gad m1 protein.

Another aspect of the invention refers to a pharmaceutical composition, preferably a diagnostic pharmaceutical composition for type I food allergy, which comprises as the active ingredient a diagnostically effective amount of the allergen extract of the invention. This pharmaceutical composition may further comprise one or more adjuvants, diluents, preservatives or mixtures thereof.

Another aspect of the invention refers to a kit, hereinafter the "kit of the invention", comprising the allergen extract of the invention. Preferably, the allergen extract of the invention is labelled or immobilized in the kit of the invention. This kit of the invention may additionally comprise other components needed for the *in vivo* or *in vitro* diagnosis of type I food allergy, such as negative and/or positive controls of an immunological response or medical instruments or devices for putting in contact the allergen extract of the invention with patient tissue/s or with an isolated biological sample derived from the patient. Preferably, the kit of the invention is a kit suitable for the diagnosis of type I food allergy, more preferably for the diagnosis of type I food allergy by the skin prick test (SPT).

Another aspect of the invention refers to the use of the allergen extract of the invention or the kit of the invention as a reactive for the diagnosis of type I food allergy. Alternative, this aspect of the invention refers to the use of the allergen extract of the invention or the kit of the invention for the manufacture of a reactive for the diagnosis of type I food allergy. Preferably, the diagnosis of type I food allergy is performed by SPT.

In a preferred embodiment of this aspect of the invention, the type I food allergy is fish allergy.

Another aspect of the invention refers to a method for the diagnosis of type I food allergy comprising putting in contact the skin of a subject with the allergen extract of the invention or the kit of the invention, analysing the presence of an immunological response in the subject and assigning the subject to the group of patients suffering from a type I food allergy when the immunological response is observed.

Another aspect of the invention refers to a method, hereinafter "the diagnosis method of the invention", for the *in vitro* diagnosis of type I food allergy comprising:
a. Putting in contact the allergen extract of the invention or the kit of the invention with an isolated biological sample of a subject,
b. analysing the presence of immunological interactions between the IgE comprised in the isolated biological sample and the type I food allergen comprised in the allergen extract or in the kit, and
c. assigning the subject to the group of patients suffering from a type I food allergy when the immunological interactions of step (b) are observed.

In a preferred embodiment, the type I food allergy is fish allergy.

In a more preferred embodiment, the isolated biological sample is serum or epithelial tissue preferably from the skin.

The "presence of immunological interactions" as defined in the diagnosis method of the invention may be analysed by any method known in the art for detecting interactions between molecules, specifically antigen-antibody interactions. Examples of such methods are, but without limitations, magnetic resonance imaging, flow cytometry, immunohistochemistry, ELISA, and/or Western Blot.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

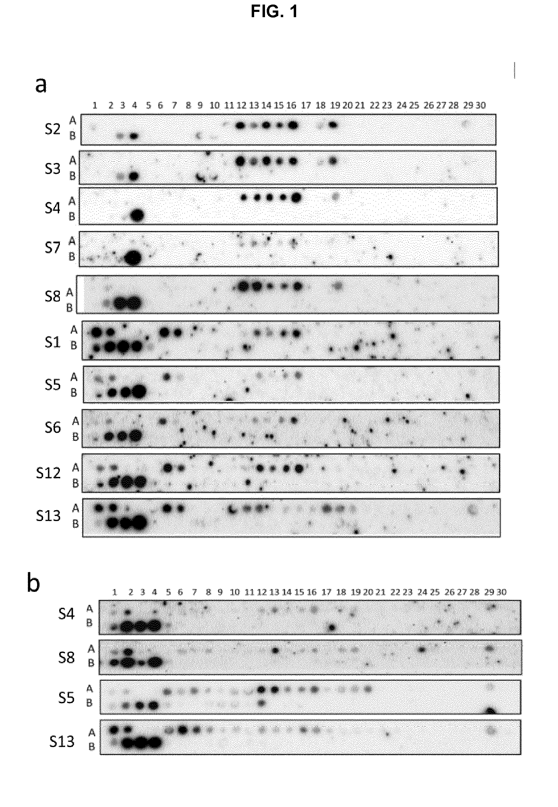

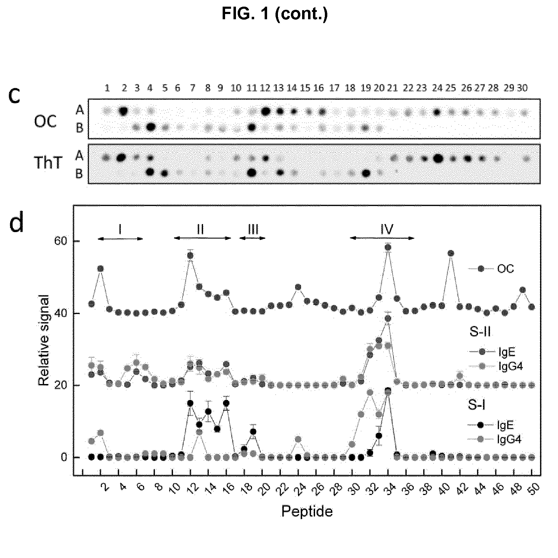

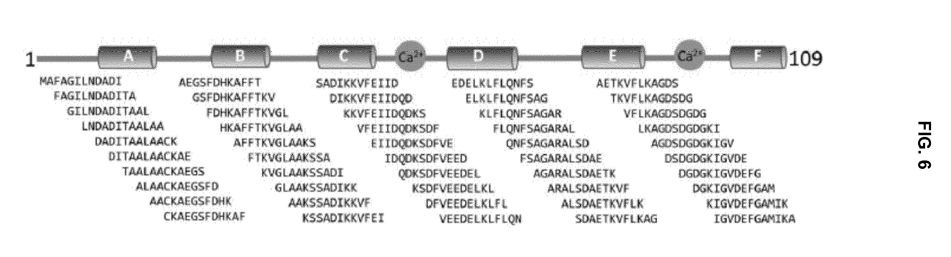

**Figure 1****. Reactivity of Gad m 1 chain. (a)** Regions binding IgE in sera from fish-allergic patients. **(b)** Regions binding IgG4 in sera from fish-allergic patients. **(c)** Sequences with anti-amyloid OC antibody reactivity and ThT binding properties. **(d)** Relative signal of the binding of IgE and IgG4 from the sera of fish-allergic patients and the anti-amyloid OC antibody to the distinct overlapping peptides representing the Gad m 1 sequence. Signals corresponding to the IgE and IgG4 binding are displayed as the average and standard deviations of the signals of sera groups S-I (S2, S3, S4, S7, and S8) and S-II (S1, S5, S6, S12, and S13) are shown. Peptides are indicated by numbers on the top (row A: peptides SEQ ID NO: 1-30, row B: peptides SEQ ID NO: 31-50) and their sequences are displayed in Fig. 6. The sera are depicted as Si, where i is a number (Table 1).

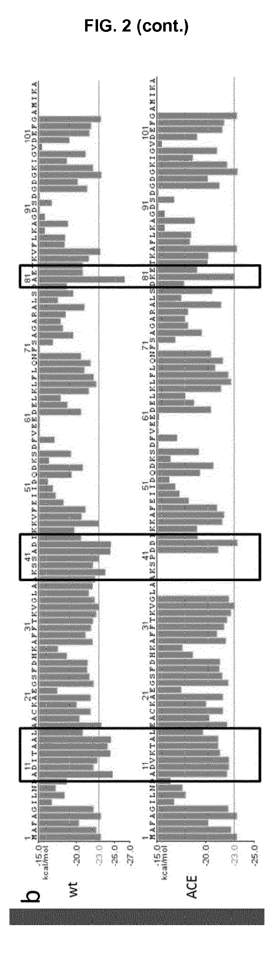

**Figure 2****. Location and relation of the immunoreactive and amyloid forming regions in the Gad m 1 chain (a)** Sequence (SEQ ID NO: 60) and location of the following regions in the Gad m 1 chain: i) IgE-binding epitopes found in sera groups S-I (S-I black and underlined) and S-II (S-II black and underlined), ii) IgG4-binding epitopes found in S-I (S-I, IgG4 grey) and S-II (S-II, IgG4 grey), iii) OC-binding segments (OC, grey), iv) adhesive regions identified by the ZipperDB algorithm (ZipperDB, grey), and v) sequence changes in the A, C and E regions (ACE, grey). The sequence changes were based on the sequences of the following β-parvalbumins: i) Q91482, Q90YK8 and E0WDA2 for A, ii) Q90YL0, Q91482, Q91483, E0WDA2 and Q90YK8 for C, and iii) C6GKU7 for E (Martinez, J. et al. Swiss Med Wkly 145, (2015)). **(b)** Comparative ZipperDB analysis of the Gad m1 wt and ACE mutant chains. The longest bars in boxes in the wt indicate the N-terminus of a hexapeptide with adhesive properties. The effects of the adhesive segments are depicted by rectangles and were conserved in the analysis of chains containing single A, C and E modifications.

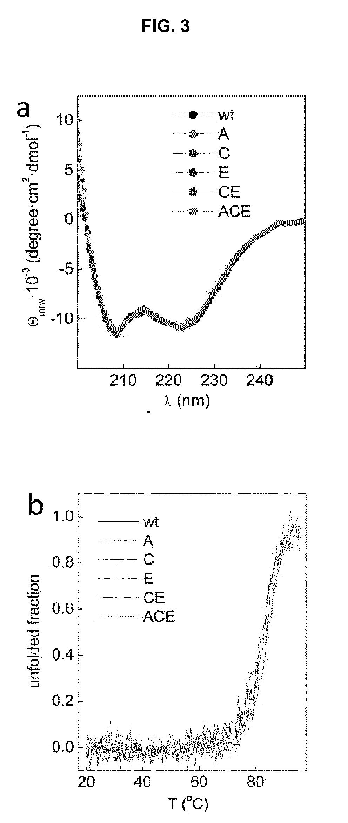

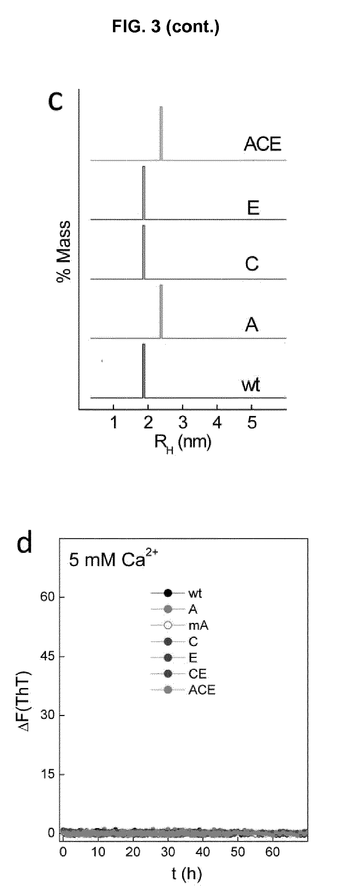

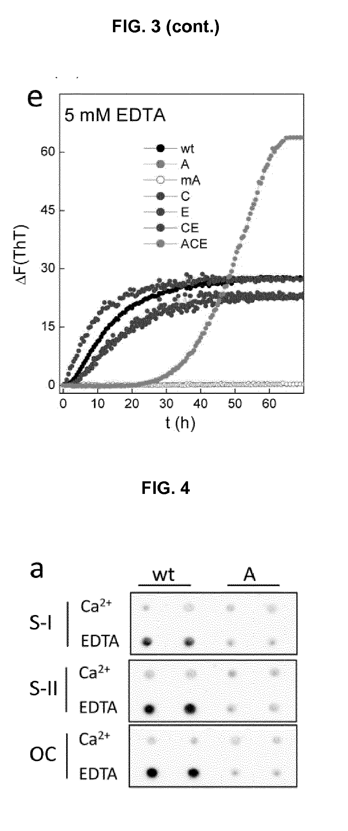

**Figure 3****. Conformational features of rGad m 1 wt and its ACE mutants. (a)** Far-V Circular Dichroism spectra of the distinct proteins obtained in 50 mM Tris-HCl, pH 7.5, containing 2 mM Ca²⁺ at 20 µM protein concentrations. **(b)** Variation of the unfolded fraction of rGad m 1 wt and mutants with temperature. **(c)** DLS determination of the hydrodynamic radius (R_{H}) of rGad m 1 wt and the mutants. The measurements were performed in 50 mM Tris-HCl, pH 7.5, containing 2 mM Ca²⁺ at 150 µM protein concentrations. Amyloid formation was monitored by the time-dependence of ThT binding of rGad m 1 wt in 50 mM Tris-HCl, pH 7.5, in the presence of **(d)** 5 mM Ca²⁺ or **(e)** 5 mM EDTA.

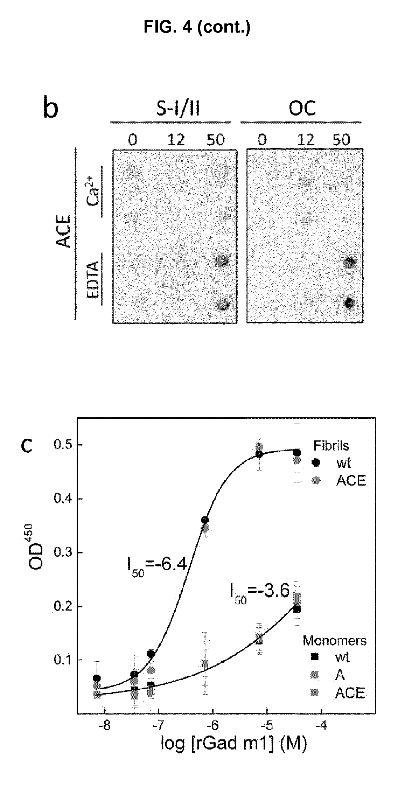

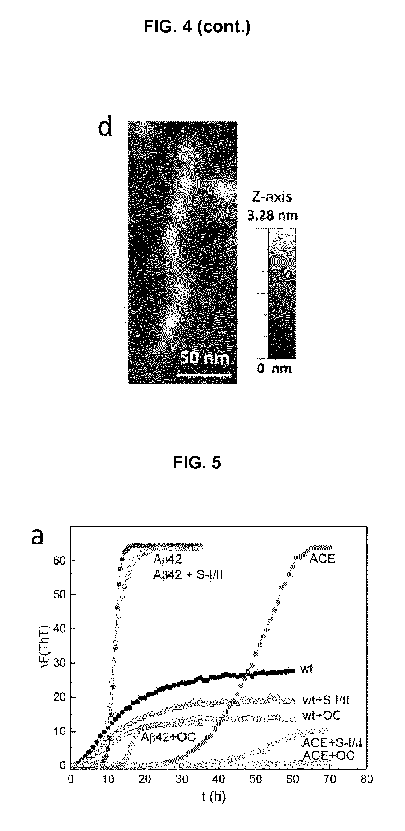

**Figure 4****. rGad m 1 amyloids displayed enhanced interaction with the IgE in the sera from fish-allergic patients. (a)** Dot-blots of rGad m wt and its non-amyloidogenic A mutant probed with the sera pools S-I and S-II and with the anti-amyloid OC antibody. Typically, 50 ng of proteins that were incubated for 50 h under monomer-preserving (5 mM Ca²⁺) and amyloid-forming (5 mM EDTA) conditions were spotted. **(b)** Dot-blots of the ACE mutant at 0, 12 and 50 h of incubation in 50 mM Tris-HCl, pH 7.5, containing 0.1 M NaCl and either 5 mM Ca²⁺ or 5 mM EDTA were probed with a sera pool (S-I/II) and the anti-amyloid OC antibody. **(c)** Titration curves of rGad m 1 wt and of its A and ACE mutants stabilized in the monomer (M) and amyloid (F) states with sera IgE generated by ELISA assays and their best fits to sigmoidal curves. The data are displayed as the mean average value of two experiments performed in duplicate and their standard deviations. **(d)** AFM image of a typical amyloid assembly of rGad m 1 and its z-axis dimension.

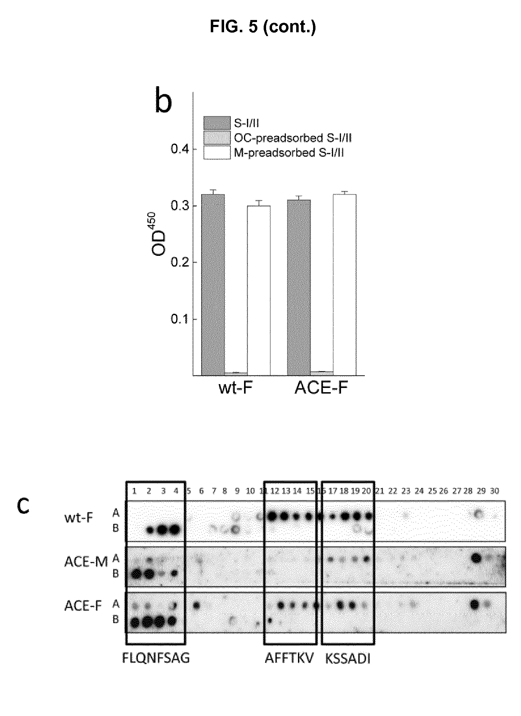

**Figure 5****. Gad m 1 sequences that are recognized by IgE in patient sera display amyloid folds. (a)** The effects of patient sera (S-I/II) and the anti-amyloid OC antibody on the amyloid formation kinetics of rGad wt, its ACE mutant and Aβ42 were monitored by ThT-binding in 50 mM Tris-HCl, pH 7.5, containing 0.1 M NaCl and 5 mM EDTA. **(b)** Effects of the anti-amyloid OC antibody and Gad m 1 monomers on recognition of the wt and ACE amyloid fibrils by the IgE in patient sera, as determined by the ELISA assay. Amyloid fibrils (100 ng/well) were probed with the patient sera pool that had been preadsorbed with 1 µg of BSA (SF), 1 µg of OC (OC-preadsorbed S-I/II) and 1 µg of the corresponding monomer chain (M-preadsorbed S-I/II). **(c)** Gad m1 peptide arrays were probed with amyloids (wt-F and ACE-F) and monomers (ACE-M) and developed with the anti-His tag antibody to detect binding. **(d)** Sequence coverage of rGad m 1 wt (SEQ ID NO: 60) obtained by LC-MS/MS analysis of the peptide mixture after pepsin digestion of the amyloids. The pepsin cleavage sites are displayed in the sequence in grey, the predicted adhesive regions are shown as continuous lines above, and the IgE binding regions are indicated with discontinuous-continuous-discontinuous lines above (solid in the common core, dotted for all peptides). The sequences of the overlapping IgE-binding regions in the peptides are depicted with continuous lines below and their mass/charge ratio (m/z) is indicated nearby.
**Figure 6****. Sequences and chain location of the 50 overlapping synthetic peptides (12 amino acid lengths, 2 amino acids offset, SEQ ID NO: 1-50) used for the identification reactive regions in Gad m 1.** Peptide numbering (1 to 50) was performed from the N- to the C-terminus of the chain sequence. Helices (A,B,C,D,E and F) forming the three EF hands are represented by rectangles, and the Ca2+ binding sites depicted as spheres.

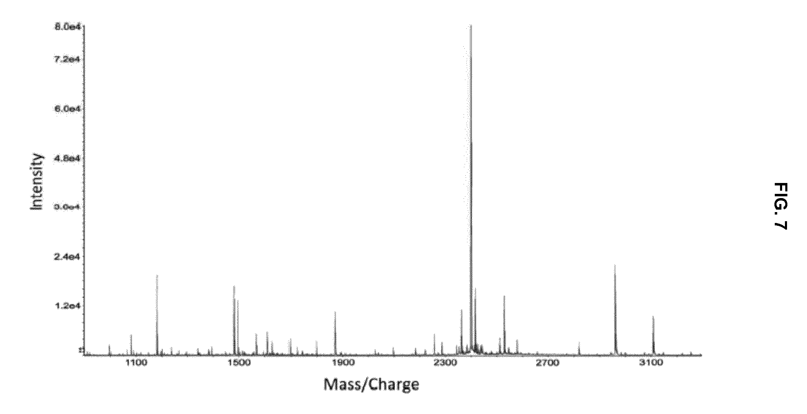

**Figure 7****. Typical MALDI-TOF/TOF spectra of pepsin-hydrolyzed rGad m 1 amyloids.** Mass spectrometry analysis was performed using α-cyano-4-hydroxycinnamic acid (CHCA) matrix. Peak features have been removed for clarity reasons.

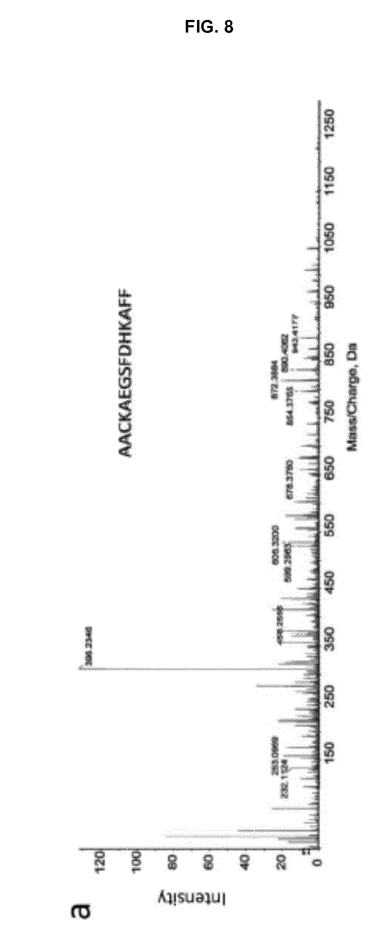

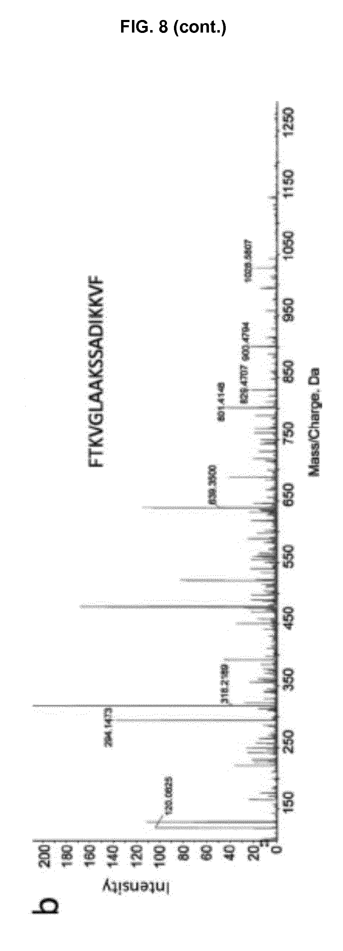

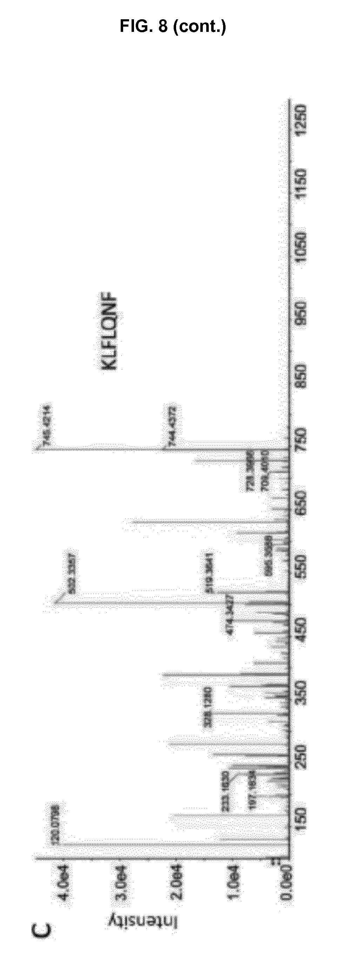

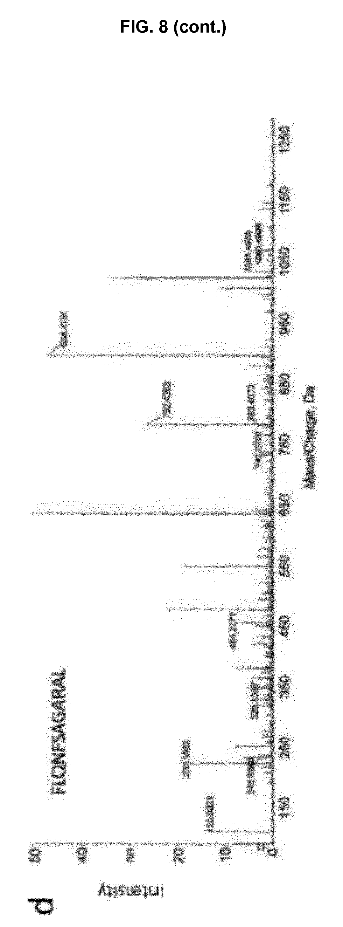

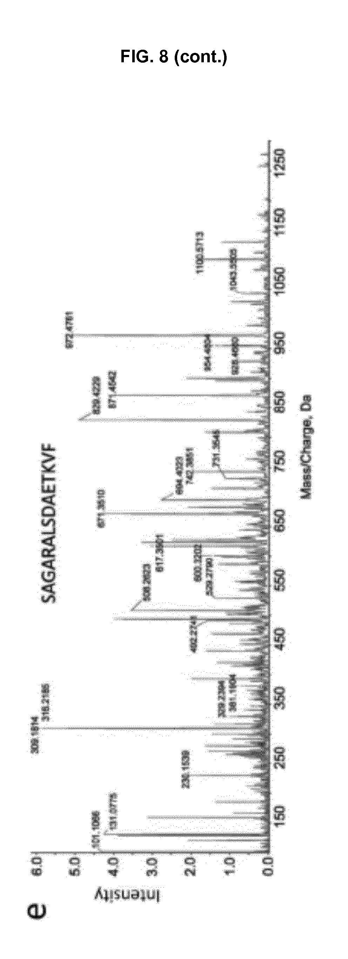

**Figure 8****. Identification of pepsin-resistant peptides of rGad m 1 amyloids.** Fragmentation spectra were obtained using nano-LC coupled to MALDI Triple TOF. Panels **(a-e)** depict the distinct sequences (SEQ ID NO: 56, 57, 58, 34 and 59) that contain undigested pepsin cleavage sites.

### EXAMPLES

It was first characterized the sequence elements to address how amyloid formation contributes to the IgE reactivity of Gad m 1. A set of 12-mer overlapping synthetic peptides with an offset of 2 that correspond to the sequence of Gad m 1 was used in an array-based immunoassay (Fig. 6).

This peptide length and surface density (10 nmol/spot) ensures the preservation of amyloid formation (which is conventionally limited to 6 residues), and differs from previous microarrays assays using a 15-mer with an offset of 3 (Perez-Gordo, M. et al. Mol Nutr Food Res 57, 1283-1290, (2013)).

The peptide membranes were assayed using the sera from ten patients who are allergic to fish (Figs. 1, 2). The IgE binding intensity was variable among the tested sera; however, it was possible to identify four major binding regions (I-IV) and two major groups of sera (S-I: S2, S3, S4, S7 and S8; S-II: S1, S5, S6, S12 and S13). Region I was only recognized by the sera group S-II and covers peptides 1-2 (SEQ ID NO: 61, FAGILNDAD common core) and 6-7 (SEQ ID NO: 62, TAALAACKAE common core). Region II, which was constituted of peptides 12-16, with SEQ ID NO: 63, FTKV as the common core, was identified by all sera with a high relative signal. In contrast, region III formed by peptides 18-19 (SEQ ID NO: 64, AAKSSADIKK common core) displayed variable recognition by the distinct sera. Region IV covering the peptides 31-34 (SEQ ID NO: 65, FLQNFS common core) displayed high intensity, but varied between the two sera groups. Of these four regions, region I overlaps the sequence predicted as the cross-β sheet forming (adhesive) segment in helix A, whereas regions III and IV overlap the immunologically reactive sites located on the junctions between the AB and CD domains (residues 33-44) and between the CD and EF domains (residues 65-74) (Martinez, J. et al. Swiss Med Wkly 145, (2015)). Nevertheless, the reactivity of the Ca²⁺-binding loop of the EF domain (residues 88-96) and of the C-terminal (residues 95-109) regions was not detected by these sera, as probed by both IgE and IgG4 binding (Fig. 1d) (Perez-Gordo, M. et al. Int Arch Allergy Imm 157, 31-40, (2012)). It merits comment that IgG4 reactivity, which is considered as a protective response in allergy, is largely detected in region IV for both sera groups (Perez-Gordo, M. et al. Int Arch Allergy Imm 157, 31-40, (2012)).

The presence of amyloid folds was then assayed using the conformation-selective OC antibody, which detects oligomer and fibril assemblies produced during the protein fibrillization process and the amyloid probe thioflavin T (ThT) (Kayed, R. et al. Molecular neurodegeneration 2, 18, (2007)). Figure 1 c shows that OC immunoreactivity covers peptides 2 (SEQ ID NO: 2, FAGILNDADITA), 12-16 (preferentially sequences containing SEQ ID NO: 63, FTKV, as the common core), 24 (SEQ ID NO: 24, VFEIIDQDKSDF), 34 (SEQ ID NO: 34, FLQNFSAGARAL), 41 (SEQ ID NO: 41, AETKVFLKAGDS) and 49 (SEQ ID NO: 49, KIGVDEFGAMIK). Of these regions, peptides 2 and 41 overlap the adhesive segments in helix A and E, predicted by the ZipperDB algorithm (Goldschmidt, L., et al. Proceedings of the National Academy of Sciences of the United States of America 107, 3487-3492, (2010)), peptides 12-16 and 34 lie over the IgE-binding regions III and IV, peptide 24 contains part of the Ca²⁺-binding site of domain CD, and peptide 49 corresponds to a previously described epitope groups (Perez-Gordo, M. et al. Mol Nutr Food Res 57, 1283-1290, (2013)) (Fig. 2 and Fig. 7). Similar to the OC reactivity, probing the membranes with ThT shows increased fluorescence emission for peptides 2, 12, 24, 34 and 41, confirming the presence of amyloid folds (Eisenberg, D. & Jucker, M. Cell 148, 1188-1203, (2012)). It must be noted that peptides containing the helix C sequence, which was predicted to be an adhesive segment, were not detected by either the OC or ThT binding assays.

Taken together, these results indicate that the Gad m 1 chain contains two major IgE binding sites (regions II and IV), which are flanked and overlapped by sequences with amyloid-forming capacity (Fig. 2a). We modified the sequences with a predicted amyloid-forming capacity identified by the ZipperDB analysis as regions A, C and E to uncouple amyloid formation from IgE binding without disrupting the linear epitopes (Figs. 2a, 3). We searched for different sequences in the A, C and E regions of β-parvalbumins from other fish species with commercial added value and tested the effect of substitutions by a second round of ZipperDB analysis to preserve the native fold (Martinez, J. et al. Swiss Med Wkly 145, (2015)). Figure 2b shows that the I12V-T13K-A14T-A17E mutations in region A, A42D-V47A mutations in region C, and E82K-F86A mutations in region E abrogate the predicted adhesive properties of each of the segments of the wt chain. All of the chains containing the single or combined mutations in regions A, C and E were produced as recombinant proteins, and their conformations were characterized. All chains yielded Ca²⁺-bound folds with predominant α-helical secondary structures and a highly stable cooperative fold with a denaturation temperature of approximately 85 °C (Fig. 3a and 3b). These data indicate that the considered single (A, C, and E mutants) and combined (ACE mutant) mutations in regions A, C and E preserve the conformation and stability of the wt protein, in agreement with their chimeric-like trend. However, the DLS analysis of their hydrodynamic features shows that the wt C and E chains yielded an R_{H} value of 1.9 ± 0.02 nm, similar to the R_{H}^{T} of a spherical monomer, whereas mutants A and ACE displayed an R_{H} value of 2.4 ± 0.04 nm, indicating a less compact fold (Fig. 3c).

Based on this conformational pattern, the capacity of the different chains to form amyloid aggregates was analyzed using the ThT binding assay (Fig. 3d and 3e). As previously shown, incubating 150 µM rGad m 1 at pH 7.5 in the presence of EDTA produces an increase in ThT fluorescence as a result of fibrillization, whereas in neutral media and in the presence of Ca²⁺, the fluorescence of ThT remains unaltered (Martinez, J. et al. Swiss Med Wkly 145, (2015)). Similar to rGad m 1 wt, the ThT fluorescence readings of all mutant chains at pH 7.5 in the presence of Ca²⁺ remained unaltered for at least 70 h (Fig. 3d). In contrast, in the presence of EDTA, the distinct rGad m 1 mutants showed altered fibrillization: mutant E showed slightly faster fibrillization, mutants C and CE showed reduced fibrillization, and fibrillization of mutant A was abolished (Fig. 3e). The impaired amyloid formation observed in the A mutant was preserved upon cleavage of the N-terminal histidine tail, excluding tag effects, as shown for the wt chain (Martinez, J. et al. Swiss Med Wkly 145, (2015)). Interestingly, the ACE mutant displays a largely retarded (lag phase of approximately 24 h) but highly cooperative fibrillization at approximately 60 h (Fig. 3e). Therefore, the adhesive region A largely governs rGad m 1 amyloid formation and its modification provides a non-amyloid-forming chain (A mutant) and a highly retarded amyloid-forming form (ACE mutant) as function of the sequences in the regions C and E.

We first used dot-blot assays to analyze the binding features of the different states to test the effect of amyloid formation on the IgE interaction (Fig. 4a and 4b). Amyloids obtained after 70 h of incubation of rGad m 1 wt in 5 mM EDTA exhibited an at least 50-fold enhancement of IgE binding compared to the Ca²⁺-bound monomer. This enhancement was not observed with the amyloid-prone A mutant under similar conditions, and it was rescued in the ACE mutant at long incubations under the assembly conditions (Fig. 4a and 4b). Therefore, the dot-blot assays support the hypothesis that amyloid assembly endows rGad m 1 with enhanced IgE-binding activity. We developed an ELISA assay to quantify the observed differences in binding. In this assay, the monomers and amyloid states of the distinct rGad m 1 chains were immobilized at varying concentrations and their IgE binding was determined using a sera pool (Fig. 4c). Under the conditions used, both the monomers and amyloids displayed similar adsorption levels, as shown by the protein concentration determinations, allowing the assignment of signal differences to the binding process. Amyloids prepared from rGad m 1 wt and the ACE mutant yielded I₅₀ values of approximately 10^{-6.4} M, whereas titration of the distinct monomers (wt, A and ACE in 5 mM Ca²⁺) resulted in I₅₀ values of approximately 10^{-3.6} M. Using I₅₀ as the apparent dissociation constant (K_{Dapp}), these values then yield K_{Dapp} of 4x10⁻⁷ M and 2x10⁻⁴ M for the amyloid and monomer forms, respectively. These data reveal that amyloid assembly provokes a 1000-fold enhancement of IgE affinity. On the other hand, using the dimensions of the rGad m 1 fibrils from the AFM images (2 nm height, 15 nm diameter and an average length of 350 nm) (Fig. 4d), the R_{H} value of the monomer from DLS measurements (Fig. 3c) and by applying simple geometrical considerations, it can be calculated that the average number of monomers per aggregate is 370. Using this aggregation number, the amyloid K_{Dapp} amounts to 1x10⁹ M, supporting a tight interaction.

The differences observed in the K_{Dapp} of the sera IgE binding to monomers and amyloids cannot simply be explained in terms of the polymer structural repetitiveness. We assayed the effect of sera on amyloid formation to analyze whether amyloid formation sculpts the architecture of nonnative epitopes, accounting for the high IgE affinity. Fig. 5a shows that sera, like the anti-amyloid OC antibody, disrupt amyloid formation in both rGad m 1 wt and ACE, as judged from the decrease in the final ThT fluorescence intensity. Sera inhibition is specific for Gad m 1 chains because the fibrillization of Aβ42 control is only diminished by the anti-amyloid OC but remains unaffected by the presence of sera. Similarly, pre-incubation of sera with OC, but not with monomers, abrogates the binding of IgE to rGad m 1 wt and ACE amyloids (Fig. 5b). Therefore, both results indicate that sera IgE and OC compete for the recognition of Gad m 1 amyloids, which agrees with the proximity and overlap of the epitopes (Fig. 2a). We exploited the properties of the amyloid formation mechanism and searched for Gad m 1 sequences involved in fibril growth to separate the contributions from proximity and overlap and rule out steric effects (Tessier, P. M. & Lindquist, S. Nature 447, 556-561, (2007)). For this purpose, amyloid fibrils formed by wt and ACE chains and the ACE monomers were incubated with the peptide arrays and binding was determined using the anti-6XHis antibody, which recognizes the tag present in the recombinant chains. Fig. 5c shows that the amyloid fibrils differentially bound to peptides 12-15 compared to the monomers, indicating that the SEQ ID NO: 66, GSFDHKAFFTKVGLAAKS sequence, is a reactive segment for amyloid growth. Because this segment matches the IgE-binding region III (Fig. 1), these results support the sequence overlap of the antigenic and amyloid-folding activities.

We took advantage of the protease resistance of amyloids to isolate and identify the assembly core and strengthen the observed functional overlap (Martinez, J. et al. Swiss Med Wkly 145, (2015)). Given the presence of several pepsin cleavage sites at the IgE-binding regions and the stability of the assemblies at pH 1.3 (Martinez, J. et al. Swiss Med Wkly 145, (2015)), the rGad m 1 wt amyloids were extensively digested with pepsin and the resulting fragments were analyzed by mass spectrometry (Figs. 7 and 8). Figure 5d shows that among the peptides detected, the sequences SEQ ID NO: 56 AACKAEGSFDHKAFF, SEQ ID NO: 57 FTKVGLAAKSSADIKKVF, SEQ ID NO: 58 KLFLQNF, SEQ ID NO: 34 FLQNFSAGARAL and SEQ ID NO: 67 SAGARALSDAETKVFL contain protected pepsin-cleavage sites and match the IgE-binding regions III and IV. Therefore, these data support the hypothesis that the sequential IgE epitopes of Gad m 1 encrypt an amyloid fold, indicating their functions as sequence-dependent conformational epitopes.

Despite the large number of sequences and 3D structures of allergens elucidated in the last few decades, the knowledge of how allergens recognize and cross-link cell-bound IgEs is still limited (Gieras, A. et al. The Journal of allergy and clinical immunology 119, 384-390, (2007)). This lack of knowledge is largely due to the complexity of allergen epitopes and their incomplete interpretation, based on the use of structures obtained from stable and ligand-bound states (Perez-Gordo, M. et al. Mol Nutr Food Res 57, 1283-1290, (2013)). For food allergens that undergo drastic environmental changes during gastrointestinal transit, these structural templates are merely snapshots of the protein life and other nonnative structures can be formed and change their interaction repertoire. One such structural state is the amyloid state that confers a polymeric trait required for multivalent interactions through the spine structure and growth properties. Amyloid formation is a sequence-dependent process and has been described for distinct food allergens, such as Bos d 5 (β-lactoglobulin), Bos d 10 (κ-casein), Bos d 12 (αs2-casein), Gad m 1 (β-parvalbumin), Gal d 2 (ovalbumin), and Gal d 4 (lysozyme), among others (Martinez, J. et al. Swiss Med Wkly 145, (2015)). Of these processes, only Gad m 1 amyloidogenesis was studied from the allergenic point of view, revealing the IgE binding capacity of the assemblies (Martinez, J. et al. Swiss Med Wkly 145, (2015)). Here, we have found that the amyloid state of Gad m 1 is essential for its ability to bind IgE; the results provided both the architecture of the epitopes and the repetitiveness required to optimize the interaction parameters, such as affinity and multivalence. This finding identifies allergens with a previously unconsidered sequence-dependent reactive conformation and adds a novel discrete function for the amyloid state.

The search for regions coding Gad m 1 amyloid assembly showed a complex chain organization. On the one hand, the ZipperDB algorithm putatively identified three regions (A, C and E) with favorable energetic fits for steric zipper formation. However, of these regions, only A and E exhibit OC-antibody binding as peptides. According to the ThT binding kinetics, region A plays a role in the initial steps of the polymerization process (namely, the nucleation step) with an outcome that is dependent on the sequences of the C and E regions. On the other hand, the regions identified as the two major IgE linear epitopes that formed part of pepsin-resistant fragments and displayed OC binding properties escaped the ZipperDB prediction analysis. The failure of the ZipperDB prediction suggests that these segments form cross-β spines that are different than the model steric zipper used as a reference in the algorithm. In fact, spines consisting of parallel β-turns resulting from β-hairpins that are distinct from the steric zipper model have been described for other amyloids (Mompean, M. et al. The journal of physical chemistry letters 6, 2608-2615, (2015)). In addition, the interdependence of the assembly process on the A, C and E sequences suggests a role for long-range effects and the possibility of cross-β spines formed by distinct β-sheets, which are not considered in the algorithm (Goldschmidt, L., et al. Proceedings of the National Academy of Sciences of the United States of America 107, 3487-3492, (2010)).

Gad m 1 amyloids are formed from the apo form and their IgE reactivity argues against the well-established Ca²⁺-binding dependence of β-parvalbumins-IgE interaction (Martinez, J. et al. Swiss Med Wkly 145, (2015)). However, this contradiction is apparent and both facts converge when the IgE interaction is analyzed in terms of the physical state of the allergen. First, the results obtained here using the wt, A and ACE chains of Gad m 1 show that the structural impact of Ca²⁺ removal is highly dependent on the chain sequence, as amyloid formation was triggered in wt and the ACE mutant, but not in the A mutant. Therefore, these data indicate that not all apo forms will form amyloids. Second, the hypoallergenic mrCyp c 1 with the two mutated Ca²⁺ binding sites exhibits a highly stable native fold that argues against its capacity to polymerize (Zuidmeer-Jongejan, L. et al. International archives of allergy and immunology 166, 41-51, (2015)). Therefore, β-parvalbumin chains that are unable to form amyloids share a diminished IgE interaction. Third, the overlap of epitopes and amyloid folds in the AB motif, which is a hot spot for conformational exchange and lacks Ca²⁺-binding properties, suggests that amyloids could also form in the presence of Ca²⁺ as a function of the chain sequence (Moraes, A. H. et al. Proteins 82, 3032-3042, (2014)). Therefore, unless specifically removed by centrifugation, β-parvalbumin solutions may contain traces of amyloid species that, as function of their analytical use, could mislead reactivity assignments.

The finding of amyloid folds for Gad m 1 IgE epitopes agrees with the unusual structural features of the Bos d 5/Fab immunocomplex (Niemi, M. et al. Structure 15, 1413-1421, (2007)). In this complex, the epitope of the milk allergen Bos d 5 was observed as a flat β-sheet resembling a monomer unit of a cross-β sheet (Niemi, M. et al. Structure 15, 1413-1421, (2007)). Nevertheless, the determination of the structure of the allergenic cross-β sheet motif will require the use of specific approaches, such as X-ray crystallography of short fragments and solid state NMR, among others (Xiao, Y. et al. Nature structural & molecular biology 22, 499-505, (2015)). Such structures will provide the identities of the β-strand segments, the number and organization of the strands in the β-sheets, and the conformations of the non-β-strand segments. This information will be essential for the identification of as yet unconsidered targets for a potential pharmacological intervention.

Both Gad m 1 monomers and amyloids bind IgE, but with significantly different affinities. The binding of sera IgE to monomers features a high K_{Dapp} (mM range). The weakness of this interaction agrees with the loose contacts observed in the Bos d 5/Fab immunocomplex structure (Niemi, M. et al. Structure 15, 1413-1421, (2007)). In contrast, the interaction of sera IgE with Gad m 1 amyloids is featured by a K_{Dapp} in the µM range, revealing a 1000-fold enhancement of the interaction strength compared to the monomer. This tightening of the interaction can be explained by several factors. From a structural point of view, the folding of the epitope into a cross-β sheet may optimize the number and nature of contacts for optimal antibody recognition. From the stoichiometry point of view, the polymer nature of amyloids permits the saturation of the two IgE binding arms, whereas a monomer does not permit saturation. Moreover, binding of the first IgE arm consists of an intermolecular reaction, whereas the occupancy of the second site involves an intramolecular reaction.

On the other hand, allergens require the existence of at least two IgE epitopes for their deleterious IgE cross-linking on the surface of effector cells. This requirement presumes the development of either two different IgE molecules for monomeric allergens or single IgE molecules for an oligomeric allergen (Sharp, M. F. & Lopata, A. L. Clinical reviews in allergy & immunology 46, 258-271, (2014)). Thus, the presentation of the allergen as an amyloid allows the development of a single IgE molecule and the optimization by enhancing the probability of the cross-linking step.

The phase transition accompanying amyloid formation has also major implications in allergy diagnosis. Clearing amyloids (either by their physical removal or by using additives that prevent their formation) from solutions used for skin prick tests may result in false negatives compared with non-risk-free oral food challenges (Sharp, M. F. & Lopata, A. L. Clinical reviews in allergy & immunology 46, 258-271, (2014)). This strategy is of particular relevance for cases in which amyloids are preferentially formed after the partial digestion of food allergens (Martinez, J. et al. Swiss Med Wkly 145, (2015)). Therefore, in light of these results, a food allergy diagnosis should be implemented for the consideration of allergen folds displaying insolubility.

### METHODS

**Ethics statement.** Approval from the Ethics Committee (PI1950) of Hospital Universitario La Paz (Madrid, Spain) was obtained. Parents signed written informed consent, and in the case of children aged 12 or older, assent from the children was also obtained. All methods were performed in accordance with the guidelines of the Hospital Universitario La Paz.

**Patient sera.** Sera samples from 10 fish-allergic patients (mean age: 9.8 years, 7 boys) from the Hospital Universitario La Paz with specific IgE antibodies to cod parvalbumin were selected. All patients had a history of symptoms suggestive of immediate hypersensitivity elicited by eating fish, with positive skin prick tests to fish extracts: cod and tuna (1000 IC/mL; Alyostal, Stallergenes, France); swordfish and salmon (1 mg/mL; Bial Aristegui); hake (1.25 mg/mL; Laboratorios LETI S.L. ) and megrim (1 mg/mL; Laboratorios LETI S.L.), as well as specific IgE antibodies to fish, as determined by the CAP-System FEIA™ (ThermoFisher) (Table 1).

**Table 1. Clinical data and sera features of the 10 patients allergic to fish.**

| **Patient** | **Age (years)** | **Sex^{a}** | **Symptoms after fish ingestion^{b}** | **Offending fish^{c}** | **Other food allergies** | **Cod SPT (mean diameter, mm)^{d}** | **Total IgE (kU/I)** | **Cod slgE (kU/l)** |
|---|---|---|---|---|---|---|---|---|
| **S1** | 12.5 | M | OAS | Hake, megrim | | 8 | 776 | 12.2 |
| **S2** | 9.1 | M | U, OAS | Hake, tuna | Seafood, tree nuts | 12 | 1020 | 13.9 |
| **S3** | 9.4 | F | U | Megrim | | 0 | 1843 | 3.4 |
| **S4** | 10.3 | F | U, AE, OAS, V | Hake, megrim | Fruits | 5 | 851 | 8.4 |
| **S5** | 8.6 | M | U, V | Hake, cod | Egg, fruits, tree nuts | 4 | 1054 | 3.7 |
| **S6** | 8.5 | M | U, AE, BS | Hake, megrim | Egg, seafood, fruits, tree nuts | 5.5 | 4277 | 7.5 |
| **S7** | 10.3 | M | V | Hake, cod, megrim | Egg, seafood, legumes, tree nuts | 6 | 2223 | 8.0 |
| **S8** | 4.6 | M | AE, U | Hake | Egg | 15 | 901 | 15.3 |
| **S12** | 16.4 | F | AN | Hake, megrim | Fruits, tree nuts | 10.5 | 516 | 10.1 |
| **S13** | 8.5 | M | OAS | Hake, megrim | Egg | 14.5 | 354 | 27.6 |

| | | | | | | | | |
|---|---|---|---|---|---|---|---|---|
| ^{a} M: male; F: female. ^{b} OAS: oral allergy syndrome; U: urticaria; AE: angioedema; V: vomiting; BS: bronchospasm; AN: anaphylaxis. ^{c} Cod: *Godus morhuo;* Hake: *Merluccius merluccius;* Megrim: *Lepidorhombus whiffiagonis;* Tuna: *Thunnus albacares.* ^{d} SPT:skin prick test. | | | | | | | | |

**Proteins and peptides.** rGad m 1 (recombinant Gad m 1) was produced from a pET15b construct containing the synthetic ORF of *Atlantic cod* parvalbumin A51874 (Martinez, J. et al. Swiss Med Wkly 145, (2015)). The mutants were prepared using Quick-Change protocols and a pair of complementary oligonucleotides (Table 2).

**Table 2. List of primers used in this study for rGad m 1 mutant generation. PV refers to the synthetic ORF of Atlantic cod parvalbumin A51874. SEQ ID NOs: 51-55 are the sequences of the primers in the order listed below.**

| **Mutation** | **Template** | **Primer (forward)** |
|---|---|---|
| **A** | pET15b-PV | 5'-GCGGATGTGAAGACGGCGCTGGAGGCGTG-3' |
| **C** | pET15b-PV | 5'-CGAAAAGCAGCGATGATATTAAGAAGGCGTTTGAAATTATTG-3' |
| **E** | pET15b-PV | 5'-GAGCGATAAGGAAACCAAGGCGTTTCTG-3' |
| **CE** | pET15b-PVC | 5'-GAGCGATAAGGAAACCAAGGCGTTTCTG-3' |
| **ACE** | pET15b-PVCE | 5'-GCGGATGTGAAGACGGCGCTGGAGGCGTG-3' |

The proteins were produced in BL21(DE3) cells and purified from the soluble fraction by Ni²⁺-affinity chromatography (GE Healthcare Life Sciences), followed by Q-Sepharose chromatography (GE Healthcare Life Sciences) (Martinez, J. et al. Swiss Med Wkly 145, (2015)). The eluted fractions were filtered using 30 kDa-pore size Amicon Ultra-15 (Merck Millipore) and extensively dialyzed against 5 mM Hepes, pH 7.5, containing 0.1 mM CaCl₂ (Martinez, J. et al. Swiss Med Wkly 145, (2015)). Before use, the proteins were centrifuged at 16,000xg for 20 min at 4 °C to remove any insoluble material. Protein concentrations were determined using the Bradford protein assay (Bio-Rad) calibrated with BSA (Sigma, A8806). Aβ42 was obtained from GenScript and used as previously described (Martinez, J. et al. PloS one 6, e27999, doi:10.1371/journal.pone.0027999 (2011)).

**Peptide arrays (SPOT).** Dodecapeptides spanning the whole sequence of Gad m 1, with an overlap of ten residues, were solid-phase synthesized and immobilized (≈10 nmol per spot) on an Amino-PEG500-UC540 sheet at the National Centre for Biotechnology (CNB-CSIC, Madrid). Before use, the membranes were rinsed with ethanol, washed three times with TBS (25 mM Tris-HCl, pH 7.5, containing 137 mM NaCl and 2.7 mM KCI), and incubated in TBS containing 1% BSA (w/v) and 2 mM EDTA for 1 h. The membranes were then probed for 2 h with sera from patients who are allergic to fish (1/10 dilution) and the anti-amyloid fibril OC antibody (AB2286 Merck Millipore, 1/2000 dilution), prepared in TBST (TBS containing 0.05% Tween-20) supplemented with 0.5% BSA (w/v) or with rGad m 1 chains (2.5 µM) in TBS. After extensive washes with TBST, a 30 min incubation with either horseradish peroxidase-labeled anti-human IgE (Abcam ab99806, 1/2000 dilution), anti-human IgG4 (Abcam ab99823, 1/4000 dilution), goat anti-rabbit IgG (Sigma, 1/5000 dilution) or anti-6X His tag® antibody (Abcam ab18184, dilution 1/1000) was performed. The signal was developed with the ECL-Western-blotting reagent (Bio-Rad) and detected with a ChemiDoc XRS instrument (Martinez, J. et al. Swiss Med Wkly 145, (2015)). When required, the membranes were regenerated by sequential incubation with TBS containing 8M urea, 1% SDS and 0.5% β-mercaptoethanol for 30 min at 55 °C and three times with acetic acid/ethanol/Milli-Q water (10:50:40). Regions were considered major allergenic epitopes when at least two overlapping peptides were involved. Signals resulting from the binding of secondary antibodies in the absence of primary antibodies were negligible under the conditions tested.

**Circular Dichroism Spectroscopy.** Circular dichroism (CD) experiments were performed using a Jasco J-820 spectropolarimeter equipped with a Peltier-controlled thermostatted cell holder. Far UV CD spectra were recorded for a 25 µM protein concentration in 50 mM Tris-HCl, pH 7.5, supplemented with either 1 mM EDTA or 1 mM CaCl₂. Thermal denaturation experiments were performed to follow the ellipticity changes at 222 nm upon heating from 15 °C to 90 °C at a 1 degree/min heating rate. Both the spectra and thermal unfolding curves were analyzed as previously described (Martinez, J. et al. Swiss Med Wkly 145, (2015)).

**Dynamic Light Scattering.** Dynamic light scattering (DLS) measurements were performed using a DynaPro spectroscatter (Wyatt Technology) with a 1.5-mm path length and a 12 µl quartz cuvette. The average of 20-25 acquisitions of buffers and protein solutions (160 µM protein concentrations) were filtered using a 0.2 µm Whatman Anodisc-3 filter. The hydrodynamic radii (R_{H}) and mass proportions (%) of the species were derived as previously described (Martinez, J. et al. Swiss Med Wkly 145, (2015)). The experiments were performed in duplicate using two different protein preparations. The theoretical hydrodynamic radius R_{H}^{T} for spherical rGad m 1 was calculated as 1.81 nm using 0.73 cm³ g⁻¹ and 0.35 g of H₂O (g protein)⁻¹ for the particle-specific volume and hydration (Martinez, J. et al. Swiss Med Wkly 145, (2015)).

**Amyloid formation assays.** The amyloid propensity and location of the segments in β-parvalbumin chains with adhesive properties was theoretically evaluated using the ZipperDB algorithm, as previously described (Martinez, J. et al. Swiss Med Wkly 145, (2015)). rGad m 1 wt and the mutants were prepared at a concentration of 150 µM in 50 mM Glycine-HCl, pH 1.6, with 100 mM NaCl or 50 mM Tris-HCl, pH 7.5, with 100 mM NaCl, both of which were supplemented with either 5 mM EDTA or 5 mM CaCl₂. The kinetics of thioflavin T (ThT) binding was monitored by bottom reading the fluorescence intensity in a POLARstar microplate reader (BMG Labtech), as previously described (Martinez, J. et al. Swiss Med Wkly 145, (2015)). The measurements were performed using 0.18 mL samples containing 10 µM ThT and 450 nm excitation and 480 nm emission filters. The measurement program consisted of 10 flashes, a reading collected every 15 min, 0.5-min of orbital shaking at 100 rpm, and the temperature controller was set to 37 °C. All measurements were collected in duplicate, and the experiments were repeated at least twice using two different protein batches. When required, the fibers were harvested from the reaction mixtures by centrifugation at 100,000xg for 1 h using an OptimaTm^{Max} Beckman ultracentrifuge. The resulting pellet and supernatant fractions were used for the protein and ThT determinations.

**Atomic force microscopy.** rGad m 1 wt fibrils were deposited onto freshly cleaved mica surfaces for 10 min, washed with H₂O, and dried with a stream of N₂. AFM images were recorded in a MultiMode Veeco microscope and analyzed using WSxM (Nanotec), as previously described (Martinez, J. et al. Swiss Med Wkly 145, (2015)).

**Dot-blot analysis.** The immunoreactivity of the rGad m 1 wt and mutant species was assessed by dot-blot analysis using the anti-amyloid fibril OC antibody (AB2286 Merck Millipore, 1:2,000 dilution) and sera from patients who are allergic to fish (1:10 dilution). Briefly, aliquots containing 50-100 ng of protein in the different states were spotted in triplicate on a nitrocellulose membrane. Immunodetection was performed by incubating the membranes with the primary antibodies for 1 h, followed by extensive washes and 30 min incubation with horseradish peroxidase-labeled either mouse monoclonal B3102E8 anti-human IgE (Abcam, diluted 1:2,000) or goat anti-rabbit IgG (diluted 1:5,000; Sigma). The signal was developed using the ECL-Western-blotting reagent (Bio-Rad) and detected with a ChemiDoc XRS instrument (Martinez, J. et al. Swiss Med Wkly 145, (2015)).

**ELISA assays.** Polystyrene 96-well plates (Costar 3590SA) were coated with 100 µl of rGad m1 chains in 0.2 M carbonate buffer, pH 9.4, by varying the concentrations from 0.1-500 µg/ml for 2 h at 37 °C. The coated wells were blocked with 1% BSA in TBS for 30 min and then incubated with 100 µl of a 1:10 dilution of the patients' serum in TBST containing 1% BSA for 2 h at room temperature. After washing with TBST, the wells were incubated with peroxidase-labeled anti human IgE (Abcam, diluted 1:2,000) for 1.5 h at room temperature. The plates were washed again and then developed with 100 µl of TMB-turbo ELISA substrate (Thermo Scientific). The reaction was stopped after 30 min with 10 µl of 2N H₂SO₄ and the optical density (OD) was measured at 450 nm using a microplate reader (Bio-Rad 3550). The assays were performed in duplicate using blocking buffer as negative control and were statistically analyzed and fitted using Origin software. A parallel analysis using the Bradford protein assay (Bio-Rad) indicated similar extent of adsorption for all proteins used.

**Protease digestion and mass spectrometry.** Typically, 50 µl of rGad m 1 fibrils (160 µM) were digested in 10 mM Gly-HCl, pH 1.3, with pepsin (1:50 w:w protease/substrate) at 37 °C for 1 h. The reactions were stopped by the addition of 1 M Tris-HCl, pH 8. After disaggregation with hexafluorisopropanol (HIFP), the hydrolyzed products were analyzed by mass spectrometry using a CHCA matrix and a MALDI-TOF/TOF 4800 (SCIEX, reflector mode) for identification and HPLC (Eksigent Technologies nanoLC Ultra 1D plus, SCIEX) coupled to a Triple TOF 5600 (SCIEX) for fragmentation at the CNB Proteomics Facility (CNB-CSIC, Madrid).

## Claims

1. A process for producing an allergen extract comprising:
a. incubating a solution comprising a type I food allergen under conditions that allow protein polymerization, and
b. recovering the solution comprising the type I food allergen in an amyloid state after step (a).

2. The process according to claim 1, wherein the type I food allergen is a β-Parvalbumin protein, preferably the Gad m1 protein.

3. The process according to claims 1 or 2, wherein the conditions that allow protein polymerization are selected from: the presence of EDTA, the presence of an acid pH, the presence of proteases or temperature changes.

4. An allergen extract that comprises a type I food allergen in an amyloid state.

5. The allergen extract according to claim 4 obtainable by the process according to any one of claims 1 to 3.

6. The allergen extract according to claims 4 or 5, wherein the type I food allergen is a β-Parvalbumin protein, preferably the Gad m1 protein.

7. A kit comprising the allergen extract according to any one of claims 4 to 6.

8. Use of the allergen extract according to any one of claims 4 to 6 or the kit according to claim 7 as a reactive for the diagnosis of type I food allergy.

9. Use according to claim 8, wherein the type I food allergy is fish allergy.

10. A method for the *in vitro* diagnosis of type I food allergy comprising:
a. Putting in contact the allergen extract according to any one of claims 4 to 6 or the kit according to claim 7 with an isolated biological sample of a subject,
b. analysing the presence of immunological interactions between the IgE comprised in the isolated biological sample and the type I food allergen comprised in the allergen extract or in the kit, and
c. assigning the subject to the group of patients suffering from a type I food allergy when the immunological interactions of step (b) are observed.

11. The method according to claim 10, wherein the type I food allergy is fish allergy.

12. The method according to claims 10 or 11, wherein the isolated biological sample is serum.
